(19) **Europäisches Patentamt / European Patent Office / Office européen des brevets**

(11) **EP 4 526 850 B1**

(12) # EUROPEAN PATENT SPECIFICATION

(45) Date of publication and mention of the grant of the patent:
**24.06.2026  Bulletin 2026/26**

(21) Application number: **23721427.5**

(22) Date of filing: **25.04.2023**

(51) International Patent Classification (IPC):
**G06T 17/00** *(2006.01)*     **A61C 7/00** *(2006.01)*
**G16H 30/00** *(2018.01)*

(52) Cooperative Patent Classification (CPC):
**G06T 17/00; G16H 30/40; G16H 40/67;**
A61C 7/002; A61C 13/0004; G06T 2210/41

(86) International application number:
**PCT/EP2023/060828**

(87) International publication number:
**WO 2023/222340 (23.11.2023 Gazette 2023/47)**

(54) **METHOD AND SYSTEM FOR DESIGNING A DENTAL APPLIANCE**

VERFAHREN UND SYSTEM FÜR DEN ENTWURF EINES ZAHNMEDIZINISCHEN GERÄTES

PROCÉDÉ ET SYSTÈME DE CONCEPTION D'UN APPAREIL DENTAIRE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **17.05.2022  EP 22173868**

(43) Date of publication of application:
**26.03.2025  Bulletin 2025/13**

(73) Proprietor: **3Shape A/S**
**1060 Copenhagen K (DK)**

(72) Inventors:
• **ASK, Erik**
  **1060 Copenhagen K (DK)**
• **HOLLENBECK, Karl-Josef**
  **1060 Copenhagen K (DK)**

(74) Representative: **Zacco Denmark A/S**
**Arne Jacobsens Allé 15**
**2300 Copenhagen S (DK)**

(56) References cited:
GB-A- 2 505 936          US-A1- 2009 244 065
US-A1- 2015 142 400

## Description

## FIELD

The disclosure relates to performing volumetric operations faster and with reduced memory requirements. In particular, the disclosure relates to a method, system, and computer readable medium for performing volumetric operations corresponding to a digital 3D data in a digital 3D volumetric space, for example, when dental appliances are digitally designed.

## BACKGROUND

Volumetric operations for digital 3D data are used in many fields of industry and for different applications. For example, volumetric operations may be used to generate a surface from a point cloud representing a 3D shape or object, to generate a modified surface from an existing surface through morphing, e.g., by copying, offsetting, smoothing, distorting, and others.

Volumetric operations such as marching cubes algorithm provide a means of converting signed distance fields to 3D polygonal meshes. The algorithm samples a signed distance field on a high-resolution grid and creates triangles within grid cells. Because the spatial level of detail of the produced 3D mesh is determined by grid resolution, marching cubes algorithm often require extensive storage and processing time. Assuming the mesh to be reconstructed is contained in a bounding box of characteristic length L, and a desired detail level is $d$, the number of grid cells in one dimension becomes $\frac{L}{d}$, and the total number of grid cells thus $\left(\frac{L}{d}\right)^3$. In many applications, $\frac{L}{d}$ may be in the order of 500 or larger.

Variants of the marching cubes algorithm have been proposed to reduce processing time and storage requirements. For example, parallelizing computations on a Graphics Processing Unit (GPU) and for using an adaptive grid represented by an octree structure, with large grid cells in regions of few surface points and smaller cells in regions with a high surface point density. Such variant, however, only creates triangles on leaf nodes in the tree structure. There is no logical extension of the principle to volumetric operations. Other proposed octree-based algorithms may include offset operations and include tiling of the volume of interest, e.g. a digital 3D model. However, even such algorithm requires the offset surface to be represented by a narrow set of geometrical primitives such as spheres and cylinders. Storti et. al. (US 2009/244065 A1, 1 October 2009) is silent about dental modeling however, it exemplifies a 3D medical data modeling, using two signed distances per each subgrid in a volumetric space.

In dentistry, there are several treatments that involve the design and construction of dental appliances such as restorations for a single tooth, some or all teeth on a jaw. Computer-aided design (CAD) of such appliances involves operations that are preferably performed in a digital 3D volumetric space. At the same time, meshes provided by dental 3D scanners have a spatial resolution that is much smaller than the size of a tooth and particularly the size of a jaw. Thus, volumetric operations for designing restorations and appliances in dental CAD are desirable, but the ones known in the art are computationally very demanding.

It is desired to an alternative approach for performing volumetric operations corresponding to a digital 3D data in a digital 3D volumetric space, for example, when dental appliances are digitally designed that overcomes at least some of the above recited limitations.

## SUMMARY

The present invention proposes a computer implemented method as in the appended claim 1. An aspect of the present disclosure is to provide a computer implemented method that performs volumetric operations faster and with reduced memory requirements. In an embodiment, this efficiency is achieved by representing, only partially, a digital 3D volumetric space of a digital 3D dental data with subgrids. It is an advantage to avoid computations in parts of a digital 3D volumetric space because execution times or memory requirements or both may be smaller. This may further be enhanced by the fact that the disclosure operates at subgrids level as opposed to individual cell level.

The disclosed method may be particularly suitable for morphing operations in a digital 3D volumetric space such as for smoothing or blockout operations, for example to digitally filling undercut areas of teeth. This makes the disclosed method particularly relevant for computer-aided design of dental appliances, especially for dental appliances that are much larger than features on the human dentition.

According to an aspect, a computer implemented method for digitally designing a dental appliance is disclosed. The method includes receiving a digital 3D dental data in a digital 3D volumetric space, computing at least two signed distances for each subgrid of a plurality of subgrids in relation to the received digital 3D dental data where each subgrid includes a plurality of cells, identifying a group of subgrids from the plurality of subgrids such that the identified group of subgrids collectively correspond to only a portion of a bounding box comprising the received digital 3D dental data. The identification of the group of subgrids is based on the at least two computed signed distances. Finally, the dental appliance is digitally designing based on the identified group of subgrids.

According to an aspect, a computer implemented method for digitally designing a dental appliance is disclosed. The method includes receiving a digital 3D dental data in a digital 3D volumetric space, computing at least two signed distances for each subgrid of a plurality of subgrids in relation to the received digital 3D dental data, each subgrid comprising a plurality of cells, identifying, based on the at least two computed signed distances, a group of subgrids from the plurality of subgrids such that the identified group of subgrids collectively correspond only to the received digital 3D dental data, and digitally designing the dental appliance based on the identified group of subgrids.

According to an aspect, a computer implemented method for digitally designing a dental appliance is disclosed. The method includes receiving a digital 3D dental data in a digital 3D volumetric space, computing at least two signed distances for each subgrid of a plurality of subgrids in relation to the received digital 3D dental data, each subgrid comprising a plurality of cells, disregarding, based on the computed at least two signed distances, a number of subgrids from the plurality of subgrids that do not comprise the 3D digital dental data, and digitally designing the dental appliance based on a group of subgrids from the plurality of subgrids not forming part of the disregarded number of subgrids.

According to an aspect, a computer implemented method for digitally designing a dental appliance is disclosed. The method includes receiving a digital 3D dental data in a digital 3D volumetric space, computing at least two signed distances for each subgrid of a plurality of subgrids in relation to the received digital 3D dental data, each subgrid comprising a plurality of cells, disregarding, based on the at least two computed signed distances, a cluster of subgrids from the subgrids corresponding to a bounding box comprising the 3D digital dental data such that the cluster of subgrids do not comprise the 3D digital dental data, and digitally designing the dental appliance based on a group of subgrids from the plurality of subgrids not forming part of the disregarded number of subgrids.

According to an aspect, a computer implemented method for digitally designing a dental appliance is disclosed. The method comprising receiving a digital 3D dental data in a digital 3D volumetric space; computing at least two signed distances for each subgrid of a plurality of subgrids in relation to the received digital 3D dental data, identifying, based on the at least two computed signed distances, a group of subgrids from the plurality of subgrids such that the identified group of subgrids collectively correspond to the received digital 3D dental data; and finally, digitally designing the dental appliance based on the identified group of subgrids.

The dental appliance may include an appliance that is configured to provide restorative treatment such as a restoration, orthodontic treatment such as clear aligners or a preventive effect such as a nightguard. Thus, the dental appliance may also include any one of a splint, a retainer, a partial denture. The dental appliance may include a first surface, i.e an outer surface, which is arranged in vicinity to facial surfaces of teeth when the user is wearing the dental appliance. The dental appliance may include a second surface, i.e. an inner surface, which is arranged in vicinity to lingual surface surfaces of teeth when the user is wearing the dental appliance.

In some examples, an identified subgrid of the identified group of subgribs may include digital 3D dental data that corresponds to both the first surface and the second surface of a dental appliance model, in this situation it is important to assign the identified subgrid to the correct surface, which may be the surface which has the shortest signed distance to the identified subgrid of the plurality of subgrid.

The computer implemented method may comprise assigning the identified group of subgrids to either a first surface or a second surface of the digital 3D dental data based on the at least two signed distances. The at least two signed distance may include a first signed distance determined between a first point of a subgrid of the identified group of subgrids and the first surface, and a second signed distance determined between a second point of a subgrid of the identified group of subgrids and the second surface. The shortest signed distance of the first signed distance and the second signed distance determines whether the identified subgrid is assigned to the first surface or the second surface of the digital 3D digital dental data.

The digital 3D dental data represents at least a portion of at least one tooth on a jaw. The digital 3D dental data of the patient's oral cavity may include one or more of surficial three-dimensional data that may be obtained from an intraoral scanner, and/or sub-surficial three-dimensional data such as obtained from a cone beam computed tomography (CBCT) scanner. The digital 3D dental data may be represented in several ways, e.g. as a point cloud or surface information by way of a 3D polygonal mesh or any other form suitable for computing signed distances in relation to a subgrid.

Receiving the digital 3D dental data may include importing, usually in response to a user selection, the digital 3D dental data from a database. The digital 3D dental data may be acquired directly by optically scanning patient's oral cavity. Optically scanning patient's oral cavity may include direct scanning of patient's oral cavity using an intraoral scanner such as TRIOS intra oral scanner from 3 Shape AS, Denmark or dental X-ray scanner. The referred dental scanners may use different imaging techniques such as focus scanning, time-of-flight, stereography, confocal scanning, triangulation, Cone Beam Computer Tomography (CBCT) techniques. Alternatively, optically scanning patient's oral cavity may include indirect scanning of patient's oral cavity by scanning a negative impression of patient's teeth or a physical dentition model that is produced based on a negative impression of patient's teeth. The physical dentition model may be scanned using desktop dental scanners such as E-series scanners from 3 Shape AS, Denmark.

The digital 3D volumetric space may be understood as a grid within which the digital 3D dental data is received, and the dental appliance is digitally designed. The plurality of subgrids is defined in the digital 3D volumetric space such that the digital 3D volumetric space includes a plurality of subgrids. This may be achieved by dividing the digital 3D volumetric space into the plurality of subgrids, wherein the plurality of subgrids may be dimensioned equally and have the same shape. Each subgrid of the plurality of subgrids may include a plurality of cells which may be dimensioned equally. The equally dimensioned cells may generally include same shape and size. The division of the digital 3D volumetric space into subgrids or cells in each subgrid may be based on a predefined value(s), which is typically defined prior to receiving the digital 3D dental data in the digital 3D volumetric space. This division or the shape and size of the plurality of subgrids and/or the plurality of cells may even be a function of the dental appliance to be digitally designed, i.e. different predefined value(s) may be used for different dental appliances. The different value(s) may be automatically selected based on indication of the dental appliance that is imported into the digital 3D volumetric space.

Each of the identified group of subgrids may include a plurality of cells, and wherein at least two signed cell distances are determined for each of the plurality of cells and based on the at least two signed cell distances a group of cells are identified, and wherein a volumetric operation is performed for each identified cells of the group of cells.

How the signed cell distances are determined for each of the plurality of cells is similar to how the signed distances of each of the plurality of subgrids are determined. The identified group of cells may be based on a cell selection criterion which is similar to the selection of the identified subgrids,

The subgrids include a shape. In one embodiment, the shape is a cube shape. This may allow to use algorithms like marching cube algorithm. However, in another embodiment, the shape is a non-cube shape such as cuboids, tetrahedra, or other shape, which may allow a variant of marching cube algorithm to be used. In some embodiments, all subgrids of the plurality of subgrids may have the same size and with same sized cells, i.e., the same number of cells in corresponding dimensions. In some embodiments, the subgrids have the same number of cells in all three dimensions. In some such embodiments, such subgrids have cells that are cubes, so that the subgrids themselves are cubes.

By applying the plurality of cells to the computer implemented method results in an even faster method for performing volumetric operations and with reduced memory requirements.

Each subgrid of the plurality of subgrids may comprise a shape that includes a plurality of corner points. Additionally, the identified group of subgrids may include a plurality of edge points along one or more edges connecting a pair of corner points of the plurality of corner points. In another embodiment, each of the plurality of subgrids comprises a plurality of edge points along one or more edges connecting a pair of corner points of the plurality of corner points. Typically, the plurality of edge points is uniformly distributed along an edge of a subgrid. The number of corner points and/ edge points per subgrid may be chosen flexibly, possibly to best match the capability of a computer's Central Processing Unit (CPU) or a Graphical Processing Unit (GPU). Powers of 2 often yield highest computational performance. For example, cube-shaped subgrids with 8 x 8 x 8 grid points may be a good choice, or 16 x 16 x 16, or other.

In an embodiment, each subgrid of the plurality of subgrids comprises a shape comprising a plurality of corner points or vertices. Additionally or alternatively, the selected group of subgrids includes a plurality of edge points along one or more edges connecting a pair of corner points of the plurality of corner points. In another embodiment, the plurality of subgrids includes a plurality of edge points along one or more edges connecting a pair of corner points of the plurality of corner points.

In different embodiments, the corner points and/ or edge points may correspond to one or more cells contained within the subgrids. In other words, corner point and/ or edge points of the subgrid may coincide with corner points of one or more cells contained within the subgrids.

In one embodiment, the plurality of edge points is uniformly distributed along an edge of a subgrid. This results in a first distance between a point and a first neighboring point, and a second distance between the point and a second neighboring point, wherein the point, first neighboring point and second neighboring point lie along an edge. In another embodiment, the plurality of edge points is non-uniformly distributed along an edge of a subgrid. This includes at least one neighboring point pair along an edge positioned at a distance that is different from another distance between another neighboring point pair positioned along the edge. In either embodiment, it is usually preferred that the neighboring grids have coincident corner points and/ or edge points to allow for continuity and computation efficiency in the generation of surface when surface construction algorithm is applied. Alternatively, non-coincident corner points and/ or edge points between neighboring grids may be used for surface construction. However, further interpolation between the surface generated in each neighboring grid is needed, resulting in reduction in desired computational efficiency.

In an embodiment, the corner points of adjacent subgrids that are of equal dimension share the same 3D coordinates, such that the edges between corner points of adjacent subgrids are coincident. Without such coincidence, additional interpolation calculations are required, resulting in a loss of computational efficiency.

Thus, in an embodiment, a volume such as digital 3D volumetric space is represented by the plurality of subgrids. Each subgrid include cells, i.e. gridcells. The grid cells in all subgrids have a uniform size and shape. Parts of the volume that are not of interest are not covered by any subgrid. In other words, the subgrids that are not of interest are disregarded from the volume, or the subgrids that are interest are selected from the volume. The plurality of subgrids therefore typically covers a volume that is smaller than the volume that would be covered by a single big grid with cells of uniform size, as conventionally known from surface construction algorithms like marching cube algorithms in the art.

In an embodiment, the method includes placing a subgrid in the digital 3D volumetric space. Such placement may include selecting subgrids, such as the group of subgrids, that at least partially such as fully cover the digital 3D dental data or are needed to perform the volumetric operations. The subgrids needed to perform the volumetric operations may include subgrids that cover the digital 3D dental data in a different state from the received state of the digital 3D dental data, the different state being related to the volumetric operation.

In an embodiment, a determination may be made that a subgrid covers the digital 3D dental data by computing the closest signed distances between grid points (e.g. corner points or edge points) of the subgrid to the digital 3D dental data. Assuming without loss of generality that positive distances refer to grid point that lie outside the digital 3D dental data. Thus, if all grid points along the edges of a subgrid are positive, then the subgrid is outside the digital 3D dental data. Intrusions smaller than grid cell length may be overlooked with the above test but are generally may not be of interest. If all signed distances for a grid are larger than any predefined parameter value (e.g. intended offset), then said candidate subgrid would be outside relevant space of the digital 3D volumetric space needed for performing the volumetric operation, and therefore no such subgrid needs to be placed for the purpose of digitally designing the dental appliance.

Each of the plurality of subgrids may include a shape that comprises at least a first point and at least a second point, and where a first signed distance is determined between the first point and the digital 3D dental data, and at least a second signed distance is determined between the at least second point and the digital 3D dental data. The at least two signed distances include the first signed distance and the second signed distance.

The shape typically includes a plurality of corner points or vertex and one or more edges connecting a pair of corner points of the plurality of corner points. The shape needs to be understood not as a visible object in the digital 3D volumetric space, but refers to a parameter that allows a surface construction algorithm, such as a marching cube algorithm or a variant thereof to be executed for the purpose of generating a surface in the digital 3D volumetric space. Thus, the shape and any aspect thereof may also be understood in relation corner points and/or edge points, for which a value may be computed. Furthermore, the corner points and edge points are identified by their respective 3D coordinates in the digital 3D volumetric space.

The first signed distance may be a minimum distance between the first point and the digital 3D dental data, and where the second signed distance may be a minimum distance between the second point and the digital 3D dental data.

The plurality of corner points and/or the plurality of edge points includes the first point and the at least second point.

The first point and the at least second point may form part of either or both of the plurality of corner points and/or the plurality of edge points.

Computing signed distances may include using a Signed Distance Function (SDF) that receives coordinates of a point (e.g. a corner point or an edge point) in the digital 3D volumetric space and returns the shortest distance between the point and the digital 3D dental data. The sign of the return value (i.e. shortest distance) indicates whether the point is inside or outside the digital 3D dental data. The point for which the signed distances is computed may include a first point, at least a second point, corner point and/ or edge point.

To illustrate computation of signed distances, following example is presented. Consider a sphere centered at the origin. Points inside the sphere will have a distance from the origin less than the radius of the sphere, points on the sphere will have a distance equal to the radius, and points outside the sphere will have distances greater than the radius. So, Signed Distance Function for a sphere centered at the origin with a radius 1 is represented as:

$$f(x, y, z) = \sqrt{x^2 + y^2 + z^2} - 1$$

For a subgrid corner point at (x=1, y=0, z=0) in the 3D volumetric space, then f(1, 0, 0) = 0 indicating that the subgrid corner point is on the surface of the sphere. For a subgrid corner point at (x=0, y=0, z=0.5) in the 3D volumetric space, then f(0,0,0.5) = - 0.5 indicating that the subgrid corner point is inside the surface with the closest point on the surface 0.5 units away. For a subgrid corner point at (x=0, y=3, z=0) in the 3D volumetric space, then f(0,3,0) = 2 indicating that the subgrid corner point is outside the surface with the closest point on the surface 2 units away.

In an embodiment, computing signed distances may comprise determining signed distances at the plurality of corner points. In another embodiment, computing signed distances may comprise determining signed distances at the plurality of edge points located at least one of the edges. In another embodiment, computing signed distances may comprise determining signed distances at the plurality of edge points located at least one of the edges and at the plurality of corner points.

In an embodiment, the method may further include determining, based on the computed signed distances, whether a corner point of the plurality of corner points and/or an edge point of the plurality of edge points is located on a first side or on a second side of a surface included in the digital 3D dental data or on the surface. The method may further include identifying the subgrids that correspond to the digital 3D dental data based on the location of the plurality of corner points and/or the plurality of edge points in relation to the digital 3D dental data. This is possible because the subgrids includes the plurality of corner points and/or the plurality of edge points for which signed distances are computed. If corner points and/or edge points of a subgrid are identified to be within or on surface of the digital 3D dental data, the subgrids is determined to be corresponding to the digital 3D dental data.

In an embodiment, the method may further include determining, based on the computed signed distances, whether a first point and/or at least a second point is located on a first side, a second side of a surface defined by the digital 3D dental data or on the surface. The method may further include identifying the subgrids that correspond to the digital 3D dental data based on the location of the first point and the at least second point in relation to the digital 3D dental data. This is possible because the subgrids includes the first point and the at least second point for which signed distances are computed. If the first point and the at least second point of a subgrid are identified to be on a first side, a second side of a surface determined by the digital 3D dental data or on the surface, the subgrids are determined to be corresponding to the digital 3D dental data. If a subgrid does not include information about a surface corresponding the digital 3D dental data, the subgrids does not correspond to the digital 3D dental data.

The computed signed distance for each of the plurality of subgrids may be determined between a first edge point of the plurality of edge points or a first corner point of the plurality of corner points and the digital 3D dental data, and between a second edge point of the plurality of edge points or a second corner point of the plurality of corner points and the digital 3D dental data.

The at least two computed signed distances for each of the plurality of subgrids may be determined between a first corner point of the plurality of corner points and the digital 3D dental data and between a second corner point of the plurality of corner points and the digital 3D dental data. The digital 3D dental data of a subgrid may include more than one surface, and in this example, it has to be determined which of the surfaces is the most relevant to the subgrid.

It is evident that identifying or disregarding a candidate subgrid, i.e. a subgrid being examined to determine whether it corresponds to the digital 3D dental model or a part of the digital 3D dental model, usually requires examining whether respective signed distances at one or more points, for example corner points and/ or edge points of the candidate subgrid, lies on a first side of a surface, a second side of the surface or on the surface defined by the digital 3D dental data. The first side is opposite to the second side.

Accordingly, for identifying a subgrib that corresponds to the digital 3D dental model a selection criterion may be determined. The selection criterion may include at least one point, such as a corner point and/ or edge point lying within or on surface of the digital 3D dental data.

The group of subgrids may be identified in different ways. The identifying of the group of subgrids may comprise identifying a subgrid when the at least two signed distances at the first point and at the at least second point meet the selection criterion. Alternatively, the identifying of the group of subgrids may comprise identifying a subgrid when the at least two signed distances at the first corner point and at the at least second corner point meet the selection criterion. Alternatively, the identifying of the group of subgrids may comprise identifying a subgrid when the at least two signed distances at the first corner point or at the first edge point and at the at least second corner point or at the at least second edge point meet the selection criterion. The selection criterion may include at least one positive signed distance and at least one negative signed distance. Having a first point and at least a second point with a positive and a negative signed criterion, respectively, indicates that the subgrid that includes the first point and the at least second point corresponds to the digital 3D dental data.

In an embodiment, identifying the group of subgrids comprises identifying a subgrid having at least two signed distances for respectively two corner points meeting the selection criterion. Additionally or alternatively, identifying the group of subgrids comprises identifying a subgrid having at least two signed distance for respectively two edge points meeting the selection criterion. In another embodiment, disregarding the cluster of subgrids comprises disregarding a subgrid having at least two signed distances for respectively two corner points failing the selection criterion. Additionally or alternatively disregarding the cluster of subgrids comprises disregarding a subgrid having at least two signed distances for respectively two edge points failing the selection criterion. The defined number of corner points and/ or edge points may range from two and onwards.

In an embodiment, the method further includes generating a bounding box comprising the digital 3D dental data. The bounding box represents a box with a measure, typically the smallest measure, within which all information of the digital 3D dental data lie. Thus, the bounding box defines a closed volume and may also be expressed by the coordinates of borders that fully encloses the digital 3D dental data. The bounding box of the digital 3D dental data may be used as a reference for placement of subgrids. Bounding boxes of the digital 3D dental data such as 3D polygonal meshes are particularly simple to compute based on known techniques.

In an embodiment, the method further includes generating a bounding box comprising the digital 3D dental data, and at least a first set of subgrids of the plurality of subgrids lying outside the bounding box. The first set of subgrids may corresponds to a new set of subgrids that relates to an expanded surface of either the first surface or the second surface. The expanded surface may be determined by adding an offset value to the digital 3D dental data that defines either the first surface or the second surface. In another embodiment, the method further includes generating a bounding box comprising the digital 3D dental data, and a second set of subgrids of the plurality of subgrids lying at partly inside the bounding box. The second set of subgrids may correspond to the digital 3D dental data of the first surface or the second surface, i.e. none expanded surfaces. The first set of subgrids and second set of subgrids may collectively represent the plurality of subgrids.

In another embodiment, the digitally designing of the dental appliance may comprise performing a volumetric operation based on at least the identified group of subgrids. In an embodiment, the digitally designing of the dental appliance may comprise performing a volumetric operation based on the received digital 3D dental data for at least the identified group of subgrids. In an embodiment, the digitally designing of the dental appliance may comprise performing a volumetric operation based on at least a defined parameter value for at least the identified group of subgrids.

In yet another embodiment, the digitally designing of the dental appliance may comprise performing volumetric operation of the received digital 3D dental data and of at least the identified group of subgrids.

In an embodiment, the volumetric operation provides a surface of the dental appliance to be designed. The surface includes 3D dental appliance data that may be determined based on volumetric operation of the received digital 3D dental data of the identified group of subgrids.

The volumetric operation may provide at least a first surface and/or at least a second surface of the dental appliance. The surface may include 3D dental appliance data that are determined based on the received digital 3D dental data of the identified group of subgrids.

The dental appliance includes a surface which may be curve shaped. A volume may be partly encircled by the surface, where the volume may be configured to receive fully or partly one or more teeth. The dental appliance may include an opening which is configured to receive the one or more teeth and the one or more teeth are arranged within the volume.

For improving the comfort of wearing the dental appliance and to make it easier to apply the dental appliance, an offset may be applied to the design of the dental appliance which results in an increase of the volume along at least a facial-to-lingual axis of the dental appliance. The facial-to-lingual axis extends between a facial and lingual surface of the one or more teeth. The increased volume will reduce the friction between the one or more teeth and the dental appliance when applying the dental appliance onto the one or more teeth. For obtaining the wanted effect by applying the offset, the computer implemented method may further comprise computing expanded signed distances for a plurality of expanded subgrids by adding an offset value to each of the computed signed distances for the identified group of subgrids. The plurality of expanded subgrids may include digital 3D dental data of the identified group of subgrids that is expanded, performing a first volumetric operation of the expanded digital 3D dental data in the plurality of expanded subgrids. Furthermore, the method may comprise determining an expanded surface based on the first volumetric operation, and the expanded surface includes expanded digital 3D surface data which corresponds to the plurality of expanded subgrids, and computing compacted signed distances for a plurality of compacted subgrids by subtracting the offset value of the expanded signed distances for the plurality of expanded subgrids, wherein the plurality of compacted subgrids includes compacted digital 3D surface data that are determined based on the expanded digital 3D surface data. Additionally, the method may comprise performing a second volumetric operation of the compacted digital 3D surface data in the plurality of compacted subgrids, providing a compacted surface based on the second volumetric operations, and the compacted surface includes compacted digital 3D surface data which corresponds to the plurality of compacted subgrids, and providing a surface of the dental appliance based on the compacted surface.

The first and the second surface may include 3D dental appliance data that are determined based on the received digital 3D dental data of the identified group of subgrids.

The dental appliance may include a first surface, i.e an outer surface, which is arranged in vicinity to facial surfaces of teeth when the user is wearing the dental appliance. The dental appliance may include a second surface, i.e. an inner surface, which is arranged in vicinity to lingual surface surfaces of teeth when the user is wearing the dental appliance. The lingual surface is closest to the tongue of the user. For improving the comfort of wearing the dental appliance and to make it easier to apply the dental appliance, an offset may be applied to the design of the dental appliance which results in an increase of the volume between the inner and outer surface. The computer implemented method may then comprise computing expanded outer signed distances for a plurality of expanded outer subgrids by adding an outer offset value to each of the computed signed distances for the identified group of subgrids. The plurality of expanded outer subgrids includes digital 3D dental data of the identified group of subgrids that is expanded. The method may further comprise computing expanded inner signed distances for a plurality of expanded inner subgrids by adding an inner offset value to each of the computed signed distances for the identified group of subgrids, wherein the plurality of expanded inner subgrids includes digital 3D dental data of the identified group of subgrids. Furthermore, the method may comprise performing a first volumetric operation of the expanded digital 3D dental data of the plurality of expanded outer subgrids and of the plurality of expanded inner subgrids.

The inner surface and the outer surface may then be determined. The method may further comprise determining an expanded outer surface and an expanded inner surface based on the first volumetric operation, and the expanded outer surface includes expanded digital 3D surface data which corresponds to the plurality of expanded outer subgrids, and the expanded inner surface includes expanded digital 3D surface data which corresponds to the plurality of expanded inner subgrids.

The expanded outer surface and the expanded inner surface may then be compressed for providing a smoothing effect of the expanded surfaces, and the compressed surfaces may then result in a dental appliance that is optimal fitted to the user's teeth and with smooth surfaces. The method may further comprise computing compacted outer signed distances for a plurality of compacted outer subgrids by subtracting the outer offset value of the expanded outer signed distances for the plurality of expanded outer subgrids, wherein the plurality of compacted outer subgrids includes compacted digital 3D surface data that are determined based on the expanded outer surface; computing compacted inner signed distances for a plurality of compacted inner subgrids by subtracting the inner offset value of the expanded inner signed distances for the plurality of expanded inner subgrids, wherein the plurality of compacted inner subgrids includes compacted digital 3D surface data that are determined based on the expanded inner surface. Then a second volumetric operation of the compacted digital 3D dental data of the plurality of compressed outer subgrids and of the plurality of compressed inner subgrids is performed. A compressed outer surface and a compressed inner surface are determined based on the second volumetric operation, and the compressed outer surface includes compressed digital 3D surface data which corresponds to the plurality of compressed outer subgrids, and the compressed inner surface includes compressed digital 3D surface data which corresponds to the plurality of compressed inner subgrids, and providing an outer surface and an inner surface of the dental appliance based on the compacted outer surface and compacted inner surface, respectively.

The first and the second surface includes 3D dental appliance data that are determined based on the received digital 3D dental data of the identified group of subgrids.

In another example, the computer implemented method may comprise computing expanded outer signed distances and expanded inner signed distances for the plurality of subgrids by adding an outer offset value and an inner offset value, respectively, to each of the computed signed distances for the identified group of subgrids. Furthermore, the method may comprise determining an expanded outer surface and an expanded inner surface based on a first volumetric operation and the digital 3D dental data of the identified group of subgrids, and the expanded outer surface and the expanded inner surface includes expanded digital 3D surface data, and computing compressed outer signed distances and compressed inner signed distances for the plurality of subgrids by subtracting an outer offset value and an inner offset value to the expanded outer signed distances and the expanded inner signed distances, respectively. Additionally, the method may comprise determining a compressed outer surface and a compressed inner surface based on a second volumetric operation and the expanded digital 3D surface data of the expanded outer surface and the expanded inner surface, respectively, and providing an outer surface and an inner surface of the dental appliance based on the compacted outer surface and compacted inner surface, respectively.

For improving the comfort of the dental appliance, the method may further comprise broadening a part of the dental appliance. The broadening may comprise determining a broadening reference point on the dental appliance by determining a maximum width of the dental appliance along a transverse axis extending from a front part to a back part of the dental appliance, and where the transverse axis is orthogonal to an insertion axis extending from an upper part to a bottom part of the dental appliance, and broadening along the transverse axis the part of the dental appliance that is between the broadening reference point and the bottom part of the dental appliance.

The broadening may be provided to a width of a free space circumference by the surface of the dental appliance.

The broadening may be provided to a width of a free space between the first surface and the second surface of the dental appliance.

The size of the plurality of subgrids may be determined based on a type of the dental appliance to be digitally designed or based on a roughness measurement of teeth of a patient. The dental appliance is customized to be worn by the patient.

The size of the plurality of subgrids may be uniformly. It is an advantage to define uniform grid size because edges created by a surface construction algorithm like marching cubes algorithm or variant thereof for neighboring grid cells may match, hence a reconstructed surface will be continuous. This is not the case with methods in the art that use varying grid cell sizes to achieve faster computations or reduced memory requirements. Another advantage of a uniform grid size is that a grid point's position may be expressed simply by a triplet of integer indices ijk. Yet another advantage is that no geometrical primitives (e.g. shapes, boxes, planes, spheres) are required to represent an offset surface.

The identified plurality of subgrids includes cells that includes same shape and same size.

The computer implemented method may be perform by a processor of a dental system. The dental system may include an intraoral scanner configured to provide digital 3D dental data to the processor. The dental system may include a computer readable medium configured to store the digital 3D dental data before forwarding the dental data to the processor. The intraoral scanner may communicate to the processor via a wired or a wireless interface. The wireless interface may be based on WIFI and/or Bluetooth. The processor may be arranged in the intraoral scanner or in a computer device external to the intraoral scanner. The computer device may be configured to communicate with a server or a cloud server for storing remotely the designed dental appliance. The storing of the designed dental appliance may include the 3D dental appliance data of a patient and personal information of the patient. Thereby, it would be possible to retrieve the designed dental information from any dental clinic having access to the server or the cloud server.

## BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:

    FIGS. 1A, 1B and 1C illustrate a 2D cross section of a 3D volumetric space including a dental object 100;

    FIGS. 2A, 2B, 2C, 2D and 2E illustrate different example on how to determine at least two signed distances;

    FIGS. 3A and 3B illustrate a bounding box including digital 3D dental data;

    FIG. 4 illustrates an example of a digitally designed dental appliance;

    FIGS. 5A, 5B and 5C illustrate an example of a method for smoothing a surface of a dental appliance;

    FIGS. 6A, 6B and 6C illustrate an example of a method for designing a dental appliance; and

    FIGS. 7A and 7B illustrates a cross-section of a broaden dental appliance.

## DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intra-oral scan data may be performed by a dental scanning system that may include an intraoral scanning device such as the TRIOS series scanners from 3 Shape A/S. The dental scanning system may include a wireless capability as provided by a wireless network unit. The scanning device may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at a number of focus plane positions along the optical axis form said stack of 2D images (also referred to herein as a sub-scan) for a given view of the object, i.e. for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled during a scanning session, such that at least some sets of sub-scans overlap at least partially, in order to enable stitching in the post-processing. The result of stitching is the digital 3D representation of a surface larger than that which can be captured by a single sub-scan, i.e. which is larger than the field of view of the 3D scanning device. Stitching, also known as registration, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the dental object and a sequence of images of the different pattern configurations are acquired by one or more cameras located at an angle relative to the projector unit.

The scanning device comprises one or more light projectors configured to generate an illumination pattern to be projected on a three-dimensional dental object during a scanning session. The light projector(s) preferably comprises a light source, a mask having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific color, or a range of different wavelengths defining a combination of colors such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask projectors, wherein the light source is placed behind a mask having a spatial pattern, whereby the light projected on the surface of the dental object is patterned. The back-lit mask projector may comprise a collimation lens for collimating the light from the light source, said collimation lens being placed between the light source and the mask. The mask may have a checkerboard pattern, such that the generated illumination pattern is a checkerboard pattern. Alternatively, the mask may feature other patterns such as lines or dots, etc.

The scanning device preferably further comprises optical components for directing the light from the light source to the surface of the dental object. The specific arrangement of the optical components depends on whether the scanning device is a focus scanning apparatus, a scanning device using triangulation, or any other type of scanning device. A focus scanning apparatus is further described in EP 2 442 720 B1 by the same applicant, which is incorporated herein in its entirety.

The light reflected from the dental object in response to the illumination of the dental object is directed, using optical components of the scanning device, towards the image sensor(s). The image sensor(s) are configured to generate a plurality of images based on the incoming light received from the illuminated dental object. The image sensor may be a high-speed image sensor such as an image sensor configured for acquiring images with exposures of less than 1/1000 second or frame rates in excess of 250 frames pr. second (fps). As an example, the image sensor may be a rolling shutter (CCD) or global shutter sensor (CMOS). The image sensor(s) may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The network unit may be configured to connect the dental scanning system to a network comprising a plurality of network elements including at least one network element configured to receive the processed data. The network unit may include a wireless network unit or a wired network unit. The wireless network unit is configured to wirelessly connect the dental scanning system to the network comprising the plurality of network elements including the at least one network element configured to receive the processed data. The wired network unit is configured to establish a wired connection between the dental scanning system and the network comprising the plurality of network elements including the at least one network element configured to receive the processed data.

The dental scanning system preferably further comprises a processor configured to generate scan data intra-oral scan data by processing the two-dimensional (2D) images acquired by the scanning device. The processor may be part of the scanning device. As an example, the processor may comprise a Field-programmable gate array (FPGA) and/or an Advanced RISC Machines (ARM) processor located on the scanning device. The scan data comprises information relating to the three-dimensional dental object. The scan data may comprise any of: 2D images, 3D point clouds, depth data, texture data, intensity data, color data, and/or combinations thereof. As an example, the scan data may comprise one or more point clouds, wherein each point cloud comprises a set of 3D points describing the three-dimensional dental object. As another example, the scan data may comprise images, each image comprising image data e.g. described by image coordinates and a timestamp (x, y, t), wherein depth information can be inferred from the timestamp. The image sensor(s) of the scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images. The depth information may be used to generate 3D point clouds comprising a set of 3D points in space, e.g., described by cartesian coordinates (x, y, z). The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp (x, y, t) or alternatively described as (x, y, z). The scanning device may be configured to transmit other types of data in addition to the scan data. Examples of data include 3D information, texture information such as infra-red (IR) images, fluorescence images, reflectance color images, x-ray images, and/or combinations thereof.

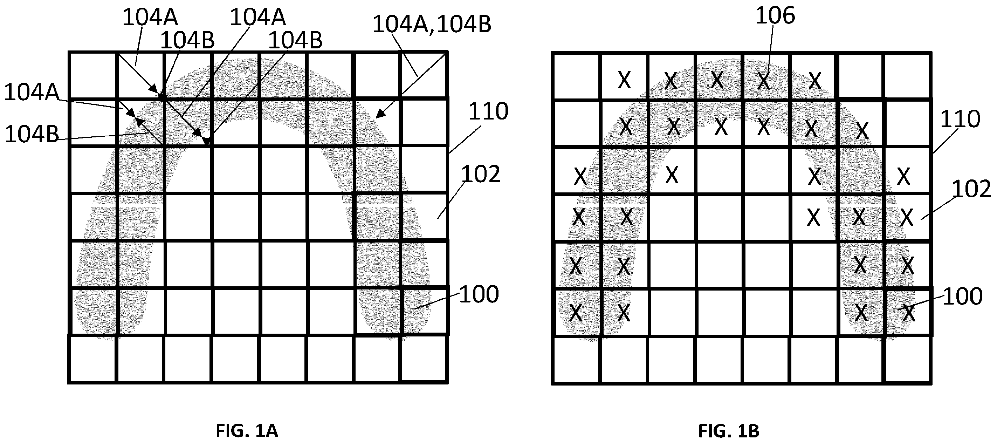

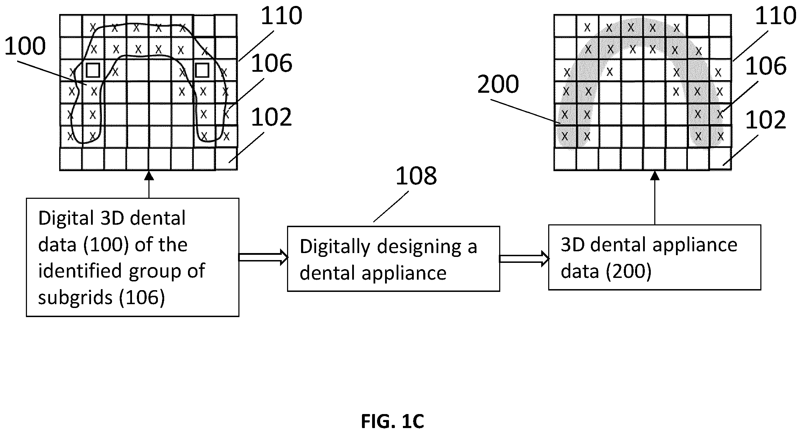

FIGS. 1A, 1B and 1C illustrate a 2D cross section within what is a 3D volumetric space 110. Accordingly, cubes are visualized as squares. FIGS. 1A and 1B illustrates further how a plurality of subgrids 102 is arranged with this invention. The 2D cross section shown is oriented orthogonal to the occlusal direction of a jaw that is the 3D dental object 100. For brevity, "dental object" is referred to as "object" in the following. In FIG. 1A, at least two signed distances (104A, 104B) are computed for each subgrid of a plurality of subgrids 102 in relation to the received digital 3D dental data, and in FIG. 1B, a group of subgrids 106 of the plurality of subgrids 106 is identified based on the at least two computed signed distances (104A,104B). The dental appliance is then digitally designed based on the identified group of subgrids 106. In present example, the plurality of subgrids is equally dimensioned including same shape and same size. In FIG. 1C the designing process 108 of a dental appliance is illustrated. The digital 3D dental data is represented by a 3D dental object 100, and of a plurality of subgrids 102 a group of subgrids 106 have been identified based on a selection criterion. A dental appliance 200 is then designed based on the group of subgrids. The designing 108 includes a smoothing of the dental appliance 200 otherwise the sharp edges applied on to the surface of the object 100 will be transferred onto the dental appliance which will be inconvenient for the user to wear. The smoothing may include expanded the digital 3D dental data and then compress the digital 3D dental data while maintaining a distance relation between points identified in each of the subgrids being part of the group of subgrids.

For brevity, "dental object" is referred to as "object" in the following.

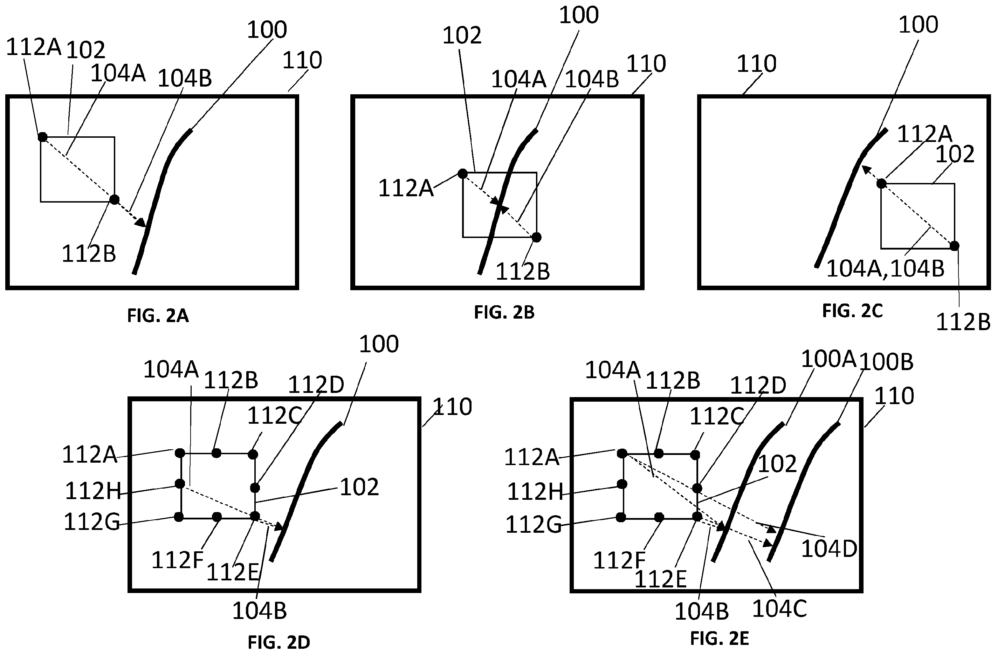

FIGS. 2A, 2B, 2C, 2D and 2E illustrate different example on how to determine the at least two signed distances (104A, 104B) of a subgrid in a digital 3D volumetric space 110 that includes the digital 3D dental data 100. In FIGS. 2A to 2C a subgrid 102 is illustrated including at least two points, respectively, a first point 112A and at least a second point 112B, and in these examples, a signed distance to the surface is determined for each of the point (112A, 112B). In FIG. 2A, the two points (112A, 112B) are located on one side of a surface 100 of the object 100 resulting in at least two signed distances (104A, 104B) being either positive or negative. In this example, the subgrid does not include a part of the object 100, i.e. digital 3D dental data 100. In FIG. 2B, the subgrid includes at least two signed distances (104A, 104B) having opposite signs, i.e. positive and negative signed distances. That indicates the subgrid 102 includes at least a part of the object 100. In FIG 2C, the at least two signed distances (104A, 104B) that have the same signs, i.e. either positive or negative. Similar to the example depicted in FIG. 2A, the subgrid 102 does not include at least a part of the object 100. In FIGS. 2A, 2B, and 2C the subgrid 102 of the plurality of subgrids includes a shape that comprises at least a first point 112A and at least a second point 112B, and where a first signed distance 104A is determined between the first point 112A and the digital 3D dental data 100, and at least a second signed distance 104B is determined between the at least second point 112B and the digital 3D dental data 100, and where the at least two signed distances include the first signed distance 104A and the second signed distance 104B. The first signed distance 104A is a minimum distance between the first point 112A and the digital 3D dental data 100, and where the second signed distance 104B is a minimum distance between the second point 112B and the digital 3D dental data 100. In the examples depicted in FIGS. 2A, 2B and 2C, the subgrid 102 includes a plurality of corner points. FIG. 2B illustrates the selection criterion of the subgrids 102 to be part of the group of subgrids. In this example, the selection criterion defines that at least two signed distances (104A, 104B) need to have different signs, such as positive and negative. In another example, the subgrid 102 could include a plurality of edge points or a combination of one or more corner points (112A, 112C, 112G, 112E) and one or more edge points (112B, 112D, 112F, 112H). That is illustrated in FIG. 2D, where the subgrid 102 includes at least two signed distances (104A, 104B) determined between a corner point 112E and the object 100 and between an edge point 112H and the object 100. In FIG. 2E, more than two signed distances (104A, 104B, 104C, 104D) are determined. In FIGS, 2A to 2E, the subgrid 102 includes uniformly distributed points (112).

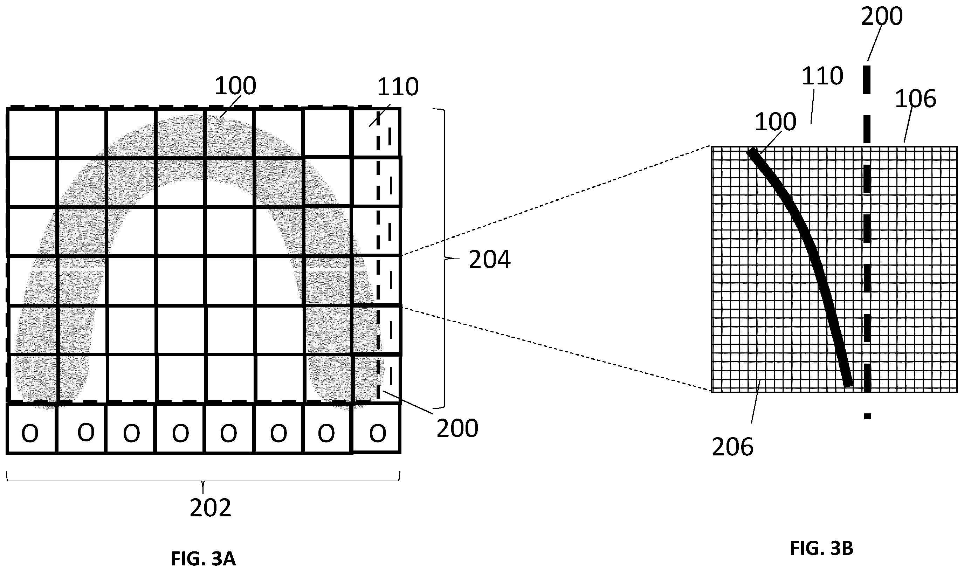

FIG. 3A illustrates a bounding box 200 that includes the digital 3D dental data 100 and a plurality of subgrids 102 in a digital 3D volumetric space 110. In this example, at least a first set of subgrids 202 of the plurality of subgrids is arranged outside the bounding box 200, and at least a second set of subgrids 204 of the plurality of subgrids is arranged partly inside the bounding box and are considered as being part of the bounding box 200. The bounding box 200 includes a third set of subgrids that is located completely within the bounding box 200. Thereby, the number of subgrids to be processed is reduced.

FIG. 3B illustrates a subgrid 106 being part of the group of subgrids that includes at least a part of the object 200. In this example, the subgrid 106 is divided into a plurality of cells 206. For each of the plurality of cells 2 at least two signed cell distances are determined, and based on the at least two signed cell distance a group of cells is identified, and wherein a volumetric operation is performed for each identified cells of the group of cells. In this specific example the cells include the same shape and size.

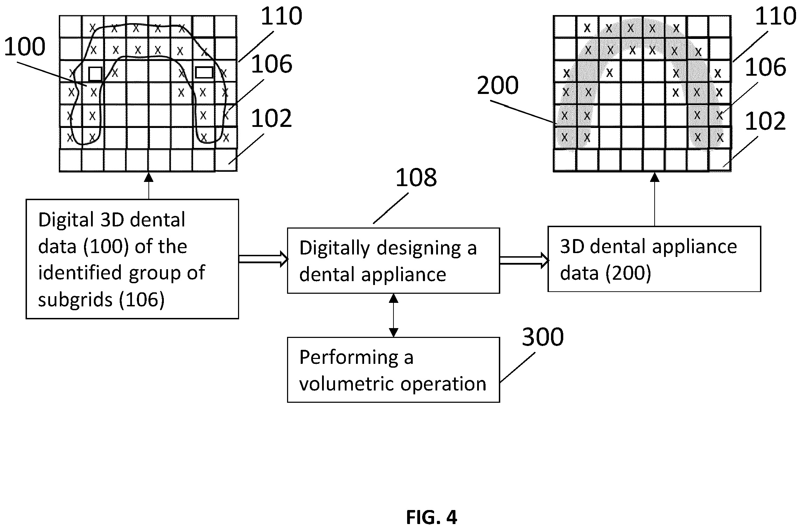

FIG 4 illustrates an example of the digitally designing of the dental appliance 200. When identified a group of subgrids 106 then a volumetric operation is performed for each of the grouped subgrids 106. As described in FIG. 3B, the volumetric operation may be even more effective in regards to processing power by defining cells within each of the identified group of subgrids 106. Thereby, the area or the amount of digital 3D dental data to be volumetric processed is reduced even more by the introduction of cells. In the design of the dental appliance 200, at least a first surface of the dental appliance 200 is designed based on the volumetric operation. In the design of the dental appliance 200, at least a second surface of the dental appliance 200 is designed based on the volumetric operation. The first surface may be an inner surface of the dental appliance 200, and the second surface may be an outer surface of the dental appliance 200. The inner surface may be arranged closer to the lingual than the outer surface.

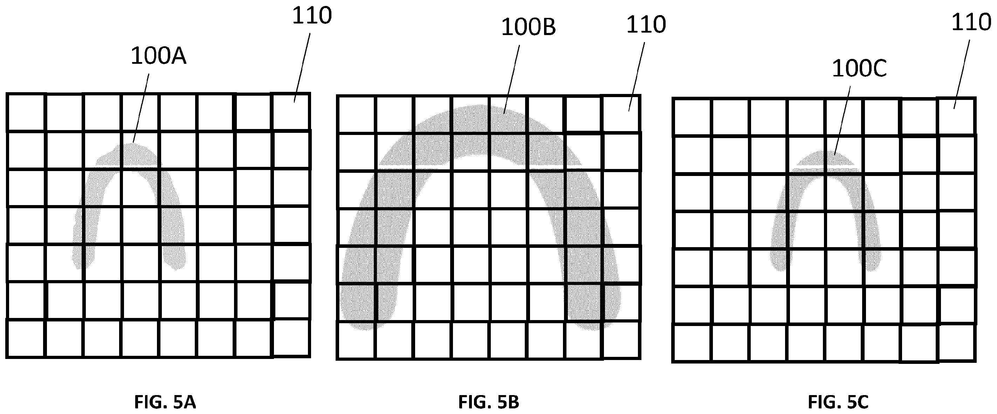

FIGS. 5A, 5B, and 5C illustrates an example of a computer implemented method for smoothing a surface of a dental appliance. FIG. 5A illustrates a digital 3D dental data 100A in a digital 3D volumetric space 110, and in FIG. 5B, an offset is added to the signed distances for the identified group of subgrids 106 resulting in an expansion of the 3D digital dental data. Based on a volumetric operation of the expanded 3D digital dental data 100B at least an expanded surface is determined. In FIG. 5B, it is seen that the expanded surface of the object 100B is smoother in comparison to the object 100A in FIG. 5A. To keep the smoothed surface when compressing the expanded surface to a size of the dental appliance that is suitable for a patients teeth/oral cavity, the offset value is subtracted from the expanded signed distances determining compacted signed distances for the identified group of subgrids 106 being compressed. The compressed group of subgrids includes compacted digital 3D surface data 100C that are determined based on the expanded digital 3D surface data 100B, and based on a second volumetric operation of the compressed digital 3D surface data 100C the surface of the dental appliance is determined, see FIG. 5C.

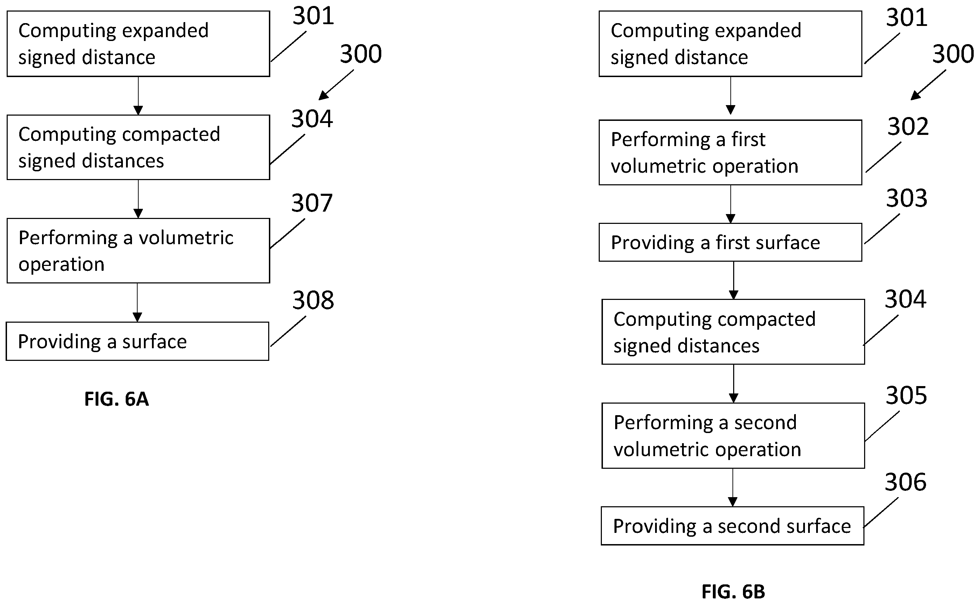

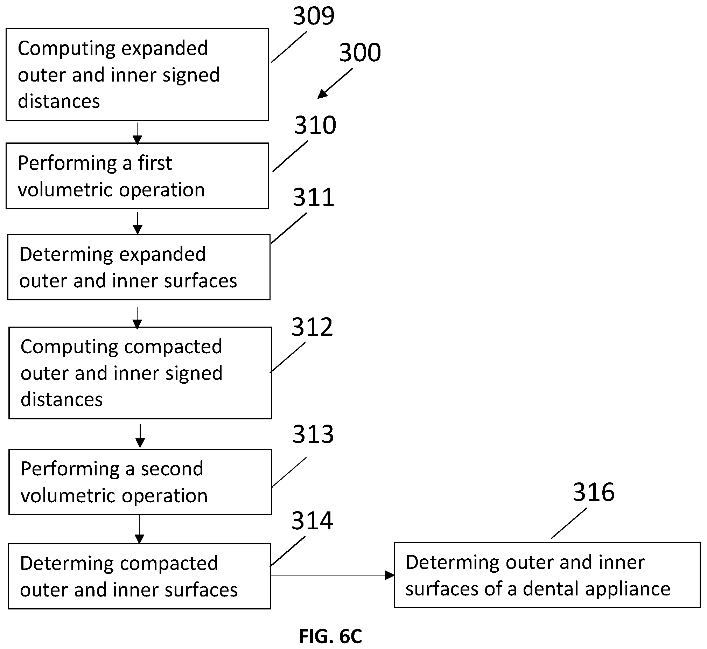

FIGS. 6A, 6B and 6C illustrates an example of a method 300 for designing a dental appliance 200. In FIG. 6A the process includes computing 301 expanded signed distances for a plurality of expanded subgrids by adding an offset value to each of the computed signed distances for the identified group of subgrids. Compacted signed distances is determined 304 for each of a plurality of compacted subgrids by subtracting the offset value of the expanded signed distances for the plurality of expanded subgrids, wherein the plurality of compacted subgrids includes compacted digital 3D surface data that are determined based on the expanded digital 3D surface data. A surface 308 of the dental appliance is determined based on a volumetric operation 307 including the plurality of compacted subgrids.

FIG. 6B illustrates another example of the method 300. The method 300 is comparable to the method described in FIG. 6A but with further steps. For example, after the expanded signed distances are determined for the plurality of expanded subgrids 301, a first volumetric operation is performed 302 of the expanded digital 3D dental data in the plurality of expanded subgrids. An expanded surface is determined 303 based on the first volumetric operation, and the expanded surface includes expanded digital 3D surface data which corresponds to the plurality of expanded subgrids. Compacted signed distances is determined 304 for a plurality of compacted subgrids by subtracting the offset value of the expanded signed distance for the plurality of expanded subgrids. The plurality of compacted subgrids includes compacted digital 3D surface data that are determined based on the expanded digital 3D surface data. A second volumetric operation is performed 305 of the compacted digital 3D surface data in the plurality of compacted subgrids. A compacted surface is determined based on the second volumetric operations, and the compacted surface includes compacted digital 3D surface data which corresponds to the plurality of compacted subgrids and providing 306 a surface of the dental appliance based on the compacted surface.

FIG. 6C illustrates yet another example of the method 300. The method includes following steps:

• Computing 309 expanded outer signed distances for a plurality of expanded outer subgrids by adding an outer offset value to each of the computed signed distances for the identified group of subgrids, wherein the plurality of expanded outer subgrids includes digital 3D dental data of the identified group of subgrids that is expanded;

• computing 309 expanded inner signed distances for a plurality of expanded inner subgrids by adding an inner offset value to each of the computed signed distances for the identified group of subgrids, wherein the plurality of expanded inner subgrids includes digital 3D dental data of the identified group of subgrids;

• performing 310 a first volumetric operation of the expanded digital 3D dental data of the plurality of expanded outer subgrids and of the plurality of expanded inner subgrids;

• determining 311 an expanded outer surface and an expanded inner surface based on the first volumetric operation, and the expanded outer surface includes expanded digital 3D surface data which corresponds to the plurality of expanded outer subgrids, and the expanded inner surface includes expanded digital 3D surface data which corresponds to the plurality of expanded inner subgrids;

• computing 312 compacted outer signed distances for a plurality of compacted outer subgrids by subtracting the outer offset value of the expanded outer signed distances for the plurality of expanded outer subgrids, wherein the plurality of compacted outer subgrids includes compacted digital 3D surface data that are determined based on the expanded outer surface;

• computing 312 compacted inner signed distances for a plurality of compacted inner subgrids by subtracting the inner offset value of the expanded inner signed distances for the plurality of expanded inner subgrids, wherein the plurality of compacted inner subgrids includes compacted digital 3D surface data that are determined based on the expanded inner surface;

• performing 313 a second volumetric operation of the compacted digital 3D dental data of the plurality of compressed outer subgrids and of the plurality of compressed inner subgrids;

• determining 314 a compressed outer surface and a compressed inner surface based on the second volumetric operation, and the compressed outer surface includes compressed digital 3D surface data which corresponds to the plurality of compressed outer subgrids, and the compressed inner surface includes compressed digital 3D surface data which corresponds to the plurality of compressed inner subgrids, and

• providing 316 an outer surface and an inner surface of the dental appliance based on the compacted outer surface and compacted inner surface, respectively.

The size of the plurality of subgrids is determined based on a type of the dental appliance to be digitally designed.

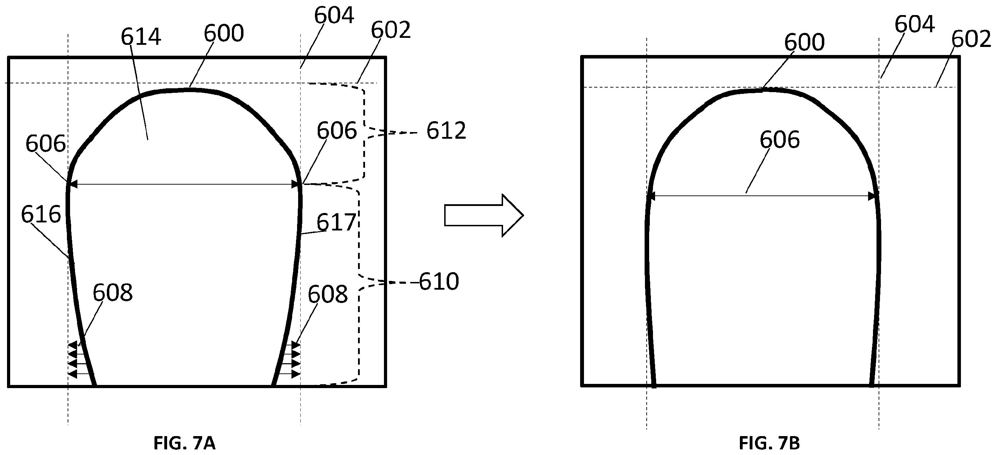

The method for designing a dental appliance includes broadening a part of the dental appliance 600 for improving the comfort of wearing the dental appliance 600, see FIGS. 7A and 7B. FIGS. 7A and 7B illustrates a cross-section of the dental appliance 600. In FIG. 7A, a broadening reference point 606 is determined on the dental appliance 600 by determining a maximum width of the dental appliance along a transverse axis 602 extending from a front part to a back part of the dental appliance 600, and where the transverse axis is orthogonal to an insertion axis 604 extending from an upper part 612 to a bottom part 610 of the dental appliance 600, and broadening along the transverse axis 602 the part of the dental appliance 600 that is between the broadening reference point 600 and the bottom part 610 of the dental appliance 600.

The broadening is provided to a width of a free space 614 circumference by the surface of the dental appliance 600.

The broadening is provided to a width of a free space between the first surface 616 and the second surface 617 of the dental appliance 600.

FIG. 7B illustrates the final dental appliance 600 after the broadening has been applied.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the present invention.

Benefits, other advantages, and solutions to problems have been described herein with regard to specific embodiments. However, the benefits, advantages, solutions to problems, and any component(s)/ unit(s) that may cause any benefit, advantage, or solution to occur or become more pronounced are not to be construed as critical, required, or essential features or components/ elements of any or all the claims or the invention. The scope of the invention is accordingly to be limited by nothing other than the appended claims, in which reference to an component/ unit/ element in the singular is not intended to mean "one and only one" unless explicitly so stated, but rather "one or more." A claim may refer to any of the preceding claims, and "any" is understood to mean "any one or more" of the preceding claims.

It is intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

**Claims**

1. A computer implemented method for digitally designing a dental appliance comprising

   receiving a digital 3D dental data in a digital 3D volumetric space, wherein the digital 3D volumetric scan includes a plurality of subgrids and wherein the digital 3D dental data includes a first surface and a second surface;
   computing at least two signed distances for each subgrid of the plurality of subgrids in relation to the received digital 3D dental data, wherein the at least two signed distances includes a first signed distance and a second signed distance, and the first signed distance is determined between a first point of a subgrid of the identified group of subgrids and the first surface, and the second signed distance is determined between a second point of a subgrid of the identified group of subgrids and the second surface, and the identifying of the group of subgrids comprises identifying a subgrid when the at least two signed distances each determined at the first point and at the at least second point meet a selection criterion, wherein the selection criterion includes that the at least two signed distances include at least one positive signed distance and at least one negative signed distance, identifying, based on the at least two computed signed distances, a group of subgrids from the plurality of subgrids such that the identified group of subgrids collectively correspond to the received digital 3D dental data; and
   digitally designing the dental appliance based on the identified group of subgrids.

2. The computer implemented method according to any of the preceding claims, further comprising defining the plurality of subgrids in the digital 3D volumetric space, wherein the plurality of subgrids is dimensioned equally.

3. The computer implemented method according to claim 2, wherein the equally dimensioned plurality of subgrids includes same shape and same size.

4. The computing implemented method according to any of the preceding claims, wherein the shortest signed distance of the first signed distance and the second signed distance determines whether the identified subgrid is assigned to the first surface or the second surface of the digital 3D digital dental data.

5. The computer implemented method according to any of the preceding claims, wherein the first signed distance is a minimum distance between the first point and the digital 3D dental data, and where the second signed distance is a minimum distance between the second point and the digital 3D dental data.

6. The computer implemented method according to any of the preceding claims, wherein each subgrid of the plurality of subgrids comprises a shape that includes a plurality of corner points, and wherein each subgrid of the plurality of subgrids comprises a plurality of edge points along one or more edges connecting a pair of corner points of the plurality of corner points, and wherein the plurality of corner points and/or the plurality of edge points includes the first point and the at least second point.

7. The computer implemented method according to claim 6, wherein the plurality of edge points is uniformly distributed along an edge of at least a subgrid of the plurality of subgrids.

8. The computer implemented method according to claim 6, wherein the at least two computed signed distances for each of the plurality of subgrids are determined between a first corner point of the plurality of corner points and the digital 3D dental data and between a second corner point of the plurality of corner points and the digital 3D dental data.

9. The computer implemented method according to claim 6, wherein the computed signed distance for each of the plurality of subgrids is determined between a first edge point of the plurality of edge points or a first corner point of the plurality of corner points and the digital 3D dental data, and between a second edge point of the plurality of edge points or a second corner point of the plurality of corner points and the digital 3D dental data.

10. The computer implemented method according to any of claims 6 to 9, wherein identifying the group of subgrids comprises identifying a subgrid when the at least two signed distances each determined at the first corner point and at the at least second corner point meet a selection criterion.

11. The computer implemented method according to any of claims 6 to 9, wherein identifying the group of subgrids comprises identifying a subgrid when the at least two signed distances each determined at the first corner point or at the first edge point and at the at least second corner point or at the at least second edge point meet a selection criterion.

12. The computer implemented method according to any of the preceding claims, comprising determining a bounding box comprising the digital 3D dental data, and at least a first set of subgrids of the plurality of subgrids that is arranged outside the bounding box, and wherein the first set of subgrids corresponds to subgrids that relate to an expanded surface of either the first surface or the second surface, wherein the expanded surface is determined by adding an offset value to the digital 3D dental data that defines either the first surface or the second surface.

13. The computer implemented method according to claim 12, comprising determining a second set of subgrids of the plurality of subgrids lying partly inside the bounding box, and wherein the second set of subgrids corresponds to the digital 3D dental data of either the first surface or the second surface.

14. The computer implemented method according to any of the preceding claims, wherein the digitally designing of the dental appliance comprises performing volumetric operation based on the received digital 3D dental data for at least the identified group of subgrids.

15. The computer implemented method according to any of preceding claims, wherein each of the identified group of subgrids includes a plurality of cells, and wherein at least two signed cell distances are determined for each of the plurality of cells, and based on the at least two signed cell distances a group of cells are identified, and wherein a volumetric operation is performed for each identified cells of the group of cells.

**Patentansprüche**

1. Computerimplementiertes Verfahren zum digitalen Entwerfen einer dentalen Vorrichtung, umfassend das Empfangen digitaler 3D-Dentaldaten in einem digitalen 3D-Volumenraum, wobei der digitale 3D-Volumenscan eine Mehrzahl von Teilgittern beinhaltet und wobei die digitalen 3D-Dentaldaten eine erste Oberfläche und eine zweite Oberfläche beinhalten;

Berechnen von mindestens zwei vorzeichenbehafteten Abständen für jedes Teilgitter der Mehrzahl von Teilgittern in Bezug auf die empfangenen digitalen 3D-Dentaldaten, wobei die mindestens zwei vorzeichenbehafteten Abstände einen ersten vorzeichenbehafteten Abstand und einen zweiten vorzeichenbehafteten Abstand beinhalten und der erste vorzeichenbehaftete Abstand zwischen einem ersten Punkt eines Teilgitters der identifizierten Gruppe von Teilgittern und der ersten Oberfläche bestimmt wird und der zweite vorzeichenbehaftete Abstand zwischen einem zweiten Punkt eines Teilgitters der identifizierten Gruppe von Teilgittern und der zweiten Oberfläche bestimmt wird, und wobei das Identifizieren der Gruppe von Teilgittern das Identifizieren eines Teilgitters umfasst, wenn die mindestens zwei vorzeichenbehafteten Abstände, die jeweils am ersten Punkt und am mindestens zweiten Punkt bestimmt werden, ein Auswahlkriterium erfüllen, wobei das Auswahlkriterium beinhaltet, dass die mindestens zwei vorzeichenbehafteten Abstände mindestens einen positiven vorzeichenbehafteten Abstand und mindestens einen negativen vorzeichenbehafteten Abstand beinhalten, Identifizieren einer Gruppe von Teilgittern aus der Mehrzahl von Teilgittern basierend auf den mindestens zwei berechneten vorzeichenbehafteten Abständen, sodass die identifizierte Gruppe von Teilgittern gemeinsam den empfangenen digitalen 3D-Dentaldaten entspricht; und

digitales Entwerfen der dentalen Vorrichtung basierend auf der identifizierten Gruppe von Teilgittern.

2. Computerimplementiertes Verfahren nach einem der vorhergehenden Ansprüche, ferner umfassend das Definieren der Mehrzahl von Teilgittern im digitalen 3D-Volumenraum, wobei die Mehrzahl von Teilgittern gleich dimensioniert ist.

3. Computerimplementiertes Verfahren nach Anspruch 2, wobei die gleich dimensionierte Mehrzahl von Teilgittern eine gleiche Form und eine gleiche Größe beinhaltet.

4. Rechnerisch implementiertes Verfahren nach einem der vorhergehenden Ansprüche, wobei der kürzeste vorzeichenbehaftete Abstand des ersten vorzeichenbehafteten Abstands und des zweiten vorzeichenbehafteten Abstands bestimmt, ob das identifizierte Teilgitter der ersten Oberfläche oder der zweiten Oberfläche der digitalen 3D-Digitaldentaldaten zugeordnet wird.

5. Computerimplementiertes Verfahren nach einem der vorhergehenden Ansprüche, wobei der erste vorzeichenbehaftete Abstand ein Mindestabstand zwischen dem ersten Punkt und den digitalen 3D-Dentaldaten ist, und wobei der zweite vorzeichenbehaftete Abstand ein Mindestabstand zwischen dem zweiten Punkt und den digitalen 3D-Dentaldaten ist.

6. Computerimplementiertes Verfahren nach einem der vorhergehenden Ansprüche, wobei jedes Teilgitter der Mehrzahl von Teilgittern eine Form umfasst, die eine Mehrzahl von Eckpunkten beinhaltet, und wobei jedes Teilgitter der Mehrzahl von Teilgittern eine Mehrzahl von Kantenpunkten entlang einer oder mehrerer Kanten beinhaltet, die ein Paar von Eckpunkten der Mehrzahl von Eckpunkten verbinden, und wobei die Mehrzahl von Eckpunkten und/oder die Mehrzahl von Kantenpunkten den ersten Punkt und den mindestens zweiten Punkt beinhaltet.

7. Computerimplementiertes Verfahren nach Anspruch 6, wobei die Mehrzahl von Kantenpunkten entlang einer Kante von mindestens einem Teilgitter der Mehrzahl von Teilgittern gleichmäßig verteilt ist.

8. Computerimplementiertes Verfahren nach Anspruch 6, wobei die mindestens zwei berechneten vorzeichenbehafteten Abstände für jedes der Mehrzahl von Teilgittern zwischen einem ersten Eckpunkt der Mehrzahl von Eckpunkten und den digitalen 3D-Dentaldaten sowie zwischen einem zweiten Eckpunkt der Mehrzahl von Eckpunkten und den digitalen 3D-Dentaldaten bestimmt werden.

9. Computerimplementiertes Verfahren nach Anspruch 6, wobei der berechnete vorzeichenbehaftete Abstand für jedes der Mehrzahl von Teilgittern zwischen einem ersten Kantenpunkt der Mehrzahl von Kantenpunkten oder einem ersten Eckpunkt der Mehrzahl von Eckpunkten und den digitalen 3D-Dentaldaten und zwischen einem zweiten Kantenpunkt der Mehrzahl von Kantenpunkten oder einem zweiten Eckpunkt der Mehrzahl von Eckpunkten und den digitalen 3D-Dentaldaten bestimmt wird.

10. Computerimplementiertes Verfahren nach einem der Ansprüche 6 bis 9, wobei das Identifizieren der Gruppe von Teilgittern das Identifizieren eines Teilgitters umfasst, wenn die mindestens zwei vorzeichenbehafteten Abstände, die jeweils am ersten Eckpunkt und am mindestens zweiten Eckpunkt bestimmt werden, ein Auswahlkriterium erfüllen.

11. Computerimplementiertes Verfahren nach einem der Ansprüche 6 bis 9, wobei das Identifizieren der Gruppe von Teilgittern das Identifizieren eines Teilgitters umfasst, wenn die mindestens zwei vorzeichenbehafteten Abstände, die jeweils am ersten Eckpunkt oder am ersten Kantenpunkt und am mindestens zweiten Eckpunkt oder am mindestens zweiten Kantenpunkt bestimmt werden, ein Auswahlkriterium erfüllen.

12. Computerimplementiertes Verfahren nach einem der vorhergehenden Ansprüche, umfassend das Bestimmen eines Begrenzungsrahmens, der die digitalen 3D-Dentaldaten beinhaltet, und mindestens eines ersten Satzes von Teilgittern der Mehrzahl von Teilgittern, der außerhalb des Begrenzungsrahmens angeordnet ist, und wobei der erste Satz von Teilgittern Teilgittern entspricht, die sich auf eine erweiterte Oberfläche entweder der ersten Oberfläche oder der zweiten Oberfläche beziehen, wobei die erweiterte Oberfläche durch Hinzufügen eines Offsetwertes zu den digitalen 3D-Dentaldaten bestimmt wird, die entweder die erste Oberfläche oder die zweite Oberfläche definieren.

**13.** Computerimplementiertes Verfahren nach Anspruch 12, umfassend das Bestimmen eines zweiten Satzes von Teilgittern der Mehrzahl von Teilgittern, die teilweise innerhalb des Begrenzungsrahmens liegen, und wobei der zweite Satz von Teilgittern den digitalen 3D-Dentaldaten entweder der ersten Oberfläche oder der zweiten Oberfläche entspricht.

**14.** Computerimplementiertes Verfahren nach einem der vorhergehenden Ansprüche, wobei das digitale Entwerfen der dentalen Vorrichtung das Durchführen einer volumetrischen Operation basierend auf den empfangenen digitalen 3D-Dentaldaten für mindestens die identifizierte Gruppe von Teilgittern umfasst.

**15.** Computerimplementiertes Verfahren nach einem der vorhergehenden Ansprüche, wobei jedes der identifizierten Gruppe von Teilgittern eine Mehrzahl von Zellen beinhaltet, und wobei mindestens zwei vorzeichenbehaftete Zellabstände für jede der Mehrzahl von Zellen bestimmt werden, und basierend auf den mindestens zwei vorzeichenbehafteten Zellabständen eine Gruppe von Zellen identifiziert wird, und wobei für jede identifizierte Zelle der Gruppe von Zellen eine volumetrische Operation durchgeführt wird.

**Revendications**

**1.** Procédé mis en œuvre par ordinateur pour la conception numérique d'un appareil dentaire, comprenant la réception de données dentaires 3D numériques dans un espace volumétrique 3D numérique, dans lequel le balayage volumétrique 3D numérique inclut une pluralité de sous-grilles et dans lequel les données dentaires 3D numériques incluent une première surface et une seconde surface ;

le calcul d'au moins deux distances signées pour chaque sous-grille de la pluralité de sous-grilles par rapport aux données dentaires 3D numériques reçues, dans lequel les au moins deux distances signées incluent une première distance signée et une seconde distance signée, et la première distance signée est déterminée entre un premier point d'une sous-grille du groupe identifié de sous-grilles et la première surface, et la seconde distance signée est déterminée entre un second point d'une sous-grille du groupe identifié de sous-grilles et la seconde surface, et l'identification du groupe de sous-grilles comprend l'identification d'une sous-grille lorsque les au moins deux distances signées, déterminées chacune au niveau du premier point et au niveau de l'au moins second point, respectent un critère de sélection, dans lequel le critère de sélection inclut le fait que les au moins deux distances signées incluent au moins une distance signée positive et au moins une distance signée négative, l'identification, sur la base des au moins deux distances signées calculées, d'un groupe de sous-grilles provenant de la pluralité de sous-grilles de sorte que le groupe identifié de sous-grilles correspond collectivement aux données dentaires 3D numériques reçues ; et la conception numérique de l'appareil dentaire sur la base du groupe identifié de sous-grilles.

**2.** Procédé mis en œuvre par ordinateur selon l'une quelconque des revendications précédentes, comprenant en outre la définition de la pluralité de sous-grilles dans l'espace volumétrique 3D numérique, dans lequel la pluralité de sous-grilles est dimensionnée de manière égale.

**3.** Procédé mis en œuvre par ordinateur selon la revendication 2, dans lequel la pluralité de sous-grilles dimensionnée de manière égale inclut une même forme et une même taille.

**4.** Procédé mis en œuvre par ordinateur selon l'une quelconque des revendications précédentes, dans lequel la distance signée la plus courte parmi la première distance signée et la seconde distance signée détermine si la sous-grille identifiée est attribuée à la première surface ou à la seconde surface des données dentaires 3D numériques.

**5.** Procédé mis en œuvre par ordinateur selon l'une quelconque des revendications précédentes, dans lequel la première distance signée est une distance minimale entre le premier point et les données dentaires 3D numériques, et dans lequel la seconde distance signée est une distance minimale entre le second point et les données dentaires 3D numériques.

**6.** Procédé mis en œuvre par ordinateur selon l'une quelconque des revendications précédentes, dans lequel chaque sous-grille de la pluralité de sous-grilles comprend une forme qui inclut une pluralité de points de coin, et dans lequel chaque sous-grille de la pluralité de sous-grilles comprend une pluralité de points d'arête le long d'une ou plusieurs arêtes reliant une paire de points de coin de la pluralité de points de coin, et dans lequel la pluralité de points de coin et/ou la pluralité de points d'arête inclut le premier point et l'au moins second point.

**7.** Procédé mis en œuvre par ordinateur selon la revendication 6, dans lequel la pluralité de points d'arête est répartie uniformément le long d'une arête d'au moins une sous-grille de la pluralité de sous-grilles.

8. Procédé mis en œuvre par ordinateur selon la revendication 6, dans lequel les au moins deux distances signées calculées pour chacune de la pluralité de sous-grilles sont déterminées entre un premier point de coin de la pluralité de points de coin et les données dentaires 3D numériques et entre un second point de coin de la pluralité de points de coin et les données dentaires 3D numériques.

9. Procédé mis en œuvre par ordinateur selon la revendication 6, dans lequel la distance signée calculée pour chacune de la pluralité de sous-grilles est déterminée entre un premier point d'arête de la pluralité de points d'arête ou un premier point de coin de la pluralité de points de coin et les données dentaires 3D numériques, et entre un second point d'arête de la pluralité de points d'arête ou un second point de coin de la pluralité de points de coin et les données dentaires 3D numériques.

10. Procédé mis en œuvre par ordinateur selon l'une quelconque des revendications 6 à 9, dans lequel l'identification du groupe de sous-grilles comprend l'identification d'une sous-grille lorsque les au moins deux distances signées, déterminées chacune au niveau du premier point de coin et au niveau de l'au moins second point de coin, respectent un critère de sélection.

11. Procédé mis en œuvre par ordinateur selon l'une quelconque des revendications 6 à 9, dans lequel l'identification du groupe de sous-grilles comprend l'identification d'une sous-grille lorsque les au moins deux distances signées, déterminées chacune au niveau du premier point de coin ou au niveau du premier point d'arête et au niveau de l'au moins second point de coin ou au niveau de l'au moins second point d'arête, respectent un critère de sélection.

12. Procédé mis en œuvre par ordinateur selon l'une quelconque des revendications précédentes, comprenant la détermination d'une boîte de délimitation comprenant les données dentaires 3D numériques, et d'au moins un premier ensemble de sous-grilles de la pluralité de sous-grilles qui est agencé à l'extérieur de la boîte de délimitation, et dans lequel le premier ensemble de sous-grilles correspond à des sous-grilles qui se rapportent à une surface étendue de soit la première surface, soit la seconde surface, dans lequel la surface étendue est déterminée en ajoutant une valeur de décalage aux données dentaires 3D numériques qui définissent soit la première surface, soit la seconde surface.

13. Procédé mis en œuvre par ordinateur selon la revendication 12, comprenant la détermination d'un second ensemble de sous-grilles de la pluralité de sous-grilles se trouvant en partie à l'intérieur de la boîte de délimitation, et dans lequel le second ensemble de sous-grilles correspond aux données dentaires 3D numériques de soit la première surface, soit la seconde surface.

14. Procédé mis en œuvre par ordinateur selon l'une quelconque des revendications précédentes, dans lequel la conception numérique de l'appareil dentaire comprend le fait de réaliser une opération volumétrique sur la base des données dentaires 3D numériques reçues pour au moins le groupe identifié de sous-grilles.

15. Procédé mis en œuvre par ordinateur selon l'une quelconque des revendications précédentes, dans lequel chaque sous-grille du groupe identifié de sous-grilles inclut une pluralité de cellules, et dans lequel au moins deux distances de cellule signées sont déterminées pour chaque cellule de la pluralité de cellules, et sur la base des au moins deux distances de cellule signées, un groupe de cellules est identifié, et dans lequel une opération volumétrique est réalisée pour chaque cellule identifiée du groupe de cellules.

**FIG. 1A**

**FIG. 1B**

EP 4 526 850 B1

100

110
106
102

200

110
106
102

108

Digital 3D dental data (100) of the identified group of subgrids (106)

Digitally designing a dental appliance

3D dental appliance data (200)

**FIG. 1C**

FIG. 2A

FIG. 2B

FIG. 2C

FIG. 2D

FIG. 2E

FIG. 3B

FIG. 3A

FIG. 4

EP 4 526 850 B1

FIG. 5A

FIG. 5B

FIG. 5C

```
┌─────────────────────┐   301
│ Computing expanded  │  ╱
│ signed distance     │        300
└─────────────────────┘      ╱
           │               ↙
           ▼                    304
┌─────────────────────┐  ╱
│ Computing compacted │
│ signed distances    │
└─────────────────────┘
           │                    307
           ▼              ╱
┌─────────────────────┐
│ Performing a volumetric │
│ operation           │
└─────────────────────┘
           │                    308
           ▼              ╱
┌─────────────────────┐
│ Providing a surface │
└─────────────────────┘

        FIG. 6A
```

```
┌─────────────────────┐   301
│ Computing expanded  │  ╱
│ signed distance     │        300
└─────────────────────┘      ╱
           │               ↙
           ▼                    302
┌─────────────────────┐  ╱
│ Performing a first  │
│ volumetric operation│
└─────────────────────┘
           │                    303
           ▼              ╱
┌─────────────────────┐
│ Providing a first surface │
└─────────────────────┘
           │                    304
           ▼              ╱
┌─────────────────────┐
│ Computing compacted │
│ signed distances    │
└─────────────────────┘
           │                    305
           ▼              ╱
┌─────────────────────┐
│ Performing a second │
│ volumetric operation│
└─────────────────────┘
           │                    306
           ▼              ╱
┌─────────────────────┐
│ Providing a second surface │
└─────────────────────┘

        FIG. 6B
```

EP 4 526 850 B1

Computing expanded outer and inner signed distances — 309

Performing a first volumetric operation — 310

Determing expanded outer and inner surfaces — 311

Computing compacted outer and inner signed distances — 312

Performing a second volumetric operation — 313

Determing compacted outer and inner surfaces — 314

Determing outer and inner surfaces of a dental appliance — 316

300

**FIG. 6C**

FIG. 7A

FIG. 7B

EP 4 526 850 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009244065 A1

- EP 2442720 B1